# EUROPEAN PATENT SPECIFICATION

(11) **EP 4 531 053 B1**
(45) Date of publication and mention of the grant of the patent: **07.10.2026**
(21) Application number: 23827398.1
(22) Date of filing: 30.05.2023
(51) Int. Cl.: G06T 11/00, G16H 30/40, G16H 30/00, G06N 3/08, G06N 3/04, A61B 6/03, A61B 5/055, A61B 6/00, A61B 5/00, G06V 10/74, G06V 10/82

(54) **ARTIFICIAL-INTELLIGENCE-BASED MEDICAL IMAGE CONVERSION METHOD**
AUF KÜNSTLICHER INTELLIGENZ BASIERENDES VERFAHREN ZUR UMWANDLUNG MEDIZINISCHER BILDER
PROCÉDÉ DE CONVERSION D'IMAGE MÉDICALE BASÉ SUR L'INTELLIGENCE ARTIFICIELLE

(30) Priority: 21.06.2022 KR 20220075856
(43) Date of publication of application: 02.04.2025
(73) Proprietor: Polestar Healthcare Co., Ltd., Seoul 06627 (KR)
(72) Inventor: YOON, Yeo Dong, Seoul 06629 (KR); LEE, Eun Chong, Yongin-si Gyeonggi-do 16812 (KR)
(74) Representative: RGTH
(86) International application number: PCT/KR2023/007337
(87) International publication number: WO 2023/249278

(56) References cited:
- KR-A- 20190 137 283
- KR-B1- 102 254 971
- KR-B1- 102 372 200
- US-A1- 2021 225 491
- LI WEN ET AL: "Magnetic resonance image (MRI) synthesis from brain computed tomography (CT) images based on deep learning methods for magnetic resonance (MR)-guided radiotherapy", QUANTITATIVE IMAGING IN MEDICINE AND SURGERY, vol. 10, no. 6, 14 April 2020 (2020-04-14), pages 1223 - 1236, XP055918953, ISSN: 2223-4292, DOI: 10.21037/qims-19-885
- PENG YINGLIN ET AL: "Magnetic resonance-based synthetic computed tomography images generated using generative adversarial networks for nasopharyngeal carcinoma radiotherapy treatment planning", RADIOTHERAPY AND ONCOLOGY, ELSEVIER, IRELAND, vol. 150, 3 July 2020 (2020-07-03), pages 217 - 224, XP086273073, ISSN: 0167-8140, [retrieved on 20200703], DOI: 10.1016/J.RADONC.2020.06.049
- LI WEN, LI YAFEN, QIN WENJIAN, LIANG XIAOKUN, XU JIANYANG, XIONG JING, XIE YAOQIN: "Magnetic resonance image (MRI) synthesis from brain computed tomography (CT) images based on deep learning methods for magnetic resonance (MR)-guided radiotherapy", QUANTITATIVE IMAGING IN MEDICINE AND SURGERY, vol. 10, no. 6, 1 June 2020 (2020-06-01), pages 1223 - 1236, XP055918953, ISSN: 2223-4292, DOI: 10.21037/qims-19-885
- PENG YINGLIN; CHEN SHUPENG; QIN AN; CHEN MEINING; GAO XINGWANG; LIU YIMEI; MIAO JINGJING; GU HUIKUAN; ZHAO CHONG; DENG XIAOWU; QI : "Magnetic resonance-based synthetic computed tomography images generated using generative adversarial networks for nasopharyngeal carcinoma radiotherapy treatment planning", RADIOTHERAPY AND ONCOLOGY, ELSEVIER, IRELAND, vol. 150, 3 July 2020 (2020-07-03), Ireland , pages 217 - 224, XP086273073, ISSN: 0167-8140, DOI: 10.1016/j.radonc.2020.06.049

## Description

### TECHNICAL FIELD

The disclosure relates to an artificial-intelligence-based medical image conversion method and, more particularly, to an artificial-intelligence-based medical image conversion method of performing conversion between medical images belonging to the same domain or performing conversion between medical images belonging to different domains using artificial intelligence (Al).

### BACKGROUND ART

The information disclosed in this section is only provided for an understanding of background information of embodiments of the disclosure and should not be taken as a description of the prior art.

Compared to magnetic resonance imaging (MRI), computed tomography (CT) imaging has the advantages of being much less expensive while having a shorter examination time and fewer restrictions on imaging artificial objects in the body, but also has the disadvantage of high radiation doses.

MRI also has the advantage of higher resolution of soft tissues, which allows for more precise tumor delineation, and because no radiation is used, the risk of radiation exposure may be minimized. However, magnetic resonance (MR) scanners are very expensive, and MRI is time consuming and expensive to perform. In some cases, MRI may not be performed if a patient has an implant in the body or is allergic to certain contrast agents.

In general, different tissues may be viewed in a non-enhanced CT image and in a contrast enhanced CT image. Different tissues may also be viewed in a CT image and in an MR image.

Therefore, if physicians are able to use both a CT image and an MR image as bases for diagnosis in clinical judgment, physicians may desirably take the best of the respective images to make an accurate diagnosis.

However, as described above, CT imaging has the problem of radiation exposure, and MRI may not be performed if an artificial object is implanted in the patient's body or the patient is allergic to contrast agents.

Therefore, if MR images may be generated based on CT images, or CT images may be generated based on MR images, physicians may not only make accurate judgments, but also accurate treatments using multimodality images, which may improve the level of medical technology in Korea and contribute to improving the health and quality of life of people.

Therefore, it is urgent to develop a technology which performs conversion between medical images belonging to different domains, such as generating MR images based on CT images or CT images based on MR images.

The information disclosed in the Background section is technical information that the inventors possessed for, or acquired during, derivation of embodiments of the disclosure and should not be taken as known technology disclosed to the public before the filing of the embodiments of the disclosure.
For example, US 2021/0225491 A1 discloses a diagnostic/medical image conversion apparatus and method in which an input CT image is converted into an MRI image by a converting module. US 2021/0225491 A1 further describes Al/GAN-based conversion using paired and/or unpaired data, with generator(s) and discriminator(s) and associated training. In particular, US 2021/0225491 A1 teaches generating a converted MRI image from a real CT image, evaluating it via a discriminator (likelihood output), calculating a likelihood loss, and generating a converted CT image back from the converted MRI image, with a reference loss computed against the real CT image, and updating network weights (e.g. via backpropagation) to minimise these losses. Furthermore, Li Wen et al., "Magnetic resonance image (MRI) synthesis from brain computed tomography (CT) images ... for MR-guided radiotherapy" (Quantitative Imaging in Medicine and Surgery, Vol. 10, No. 6, 1 June 2020) discloses synthesising brain MRI images from corresponding planning CT images using deep learning models, including GAN-based approaches (e.g. CycleGAN and Pix2Pix) and supervised architectures (e.g. U-Net). The quality of the synthesised MRI images is assessed using quantitative image similarity metrics such as MAE, MSE, SSIM and PSNR, and the use of synthetic MRI is discussed in the context of radiotherapy workflows (for example, to support image alignment/registration). Peng Yinglin et al., "Magnetic resonance-based synthetic computed tomography images generated using generative adversarial networks for nasopharyngeal carcinoma radiotherapy treatment planning" (Radiotherapy and Oncology, Vol. 150, 3 July 2020) describes generating MR-based synthetic CT images using generative adversarial networks for radiotherapy treatment planning of nasopharyngeal carcinoma.

### DISCLOSURE

### Technical Problem

Accordingly, an aspect of the disclosure has been proposed to solve the above-described problems occurring in the related art, and an objective of the disclosure is to provide a medical image conversion method and device for performing conversion between medical images belonging to different domains. For example, a medical image conversion method and device able to generate an MR image based on a CT image and, conversely, a CT image based on an MR image may be provided.

Also provided is an artificial intelligence (AI) learning model which performs conversion between medical images belonging to different domains. For example, an Al learning model able to generate an MR image based on a CT image and, conversely, a CT image based on MR an image may be provided.

The objectives of the disclosure are not limited to the aforementioned description, and other objectives not explicitly disclosed herein will be clearly understood by a person having ordinary knowledge in the art from the description provided hereinafter.

### Technical Solution

In order to achieve the above objectives, according to an aspect of the disclosure, provided is a medical image conversion method in which an artificial intelligence (AI) learning part performs conversion between medical images of different domains while performing learning. The medical image conversion method includes:
receiving, by the Al learning part, a first medical image; generating a second medical image from the first medical image, the domain of the second medical image being different from the domain of the first medical image;
generating a third medical image from the second medical image, the domain of the third medical image being different from the domain of the second medical image; and
calculating a loss value between the third medical image and the first medical image by comparing the third medical image and the first medical image.

According to embodiments, in the operation of calculating the loss value, the Al learning part may repeatedly perform the calculation of the loss value in each iteration when performing the learning.

According to embodiments, the iteration may include a first iteration and a second iteration which are sequential, and if the absolute value of a change between a first loss value calculated in the first iteration and a second loss value calculated in the second iteration is less than a threshold value, the Al learning part may stop the learning.

According to embodiments, the first medical image and the third medical image may belong to the same domain.

According to embodiments, in the operation of receiving the first medical image, the first medical image may include lesion data and normal data.

According to embodiments, if the lesion data is smaller than the normal data, the lesion data may be amplified so that the ratio of the lesion data is equal to the ratio of the normal data.

According to embodiments, if the first medical image has both a first slice for a target region and a second slice for a non-target region, the first medical image may be processed so that the second slice is removed and the first slice remains.

According to embodiments, the medical image conversion method may further include, if the first medical image is one of a pair of paired data, calculating a match rate of the second medical image and another of the pair of paired data by comparing the second medical image with the other of the pair of paired data.

According to embodiments, the medical image conversion method may further include if one of the first medical image or the second medical image which belongs to a different domain from the first medical image and is captured from the same region as the first medical image is input as the first medical image, calculating the match rate by comparing the second medical image and the second original image.

According to embodiments, the match rate may be calculated using the Hausdorff distance or the Dice similarity coefficient.

According to embodiments, if the second medical image and the other of the pair of paired data each contain a lesion region, the match rate may be calculated quantitatively using the difference in the center of mass of the locations of the two lesions.

According to another aspect of the disclosure, provided is a medical image conversion method in which an Al learning part performs conversion between medical images of different domains while performing learning. The medical image conversion method includes at least one of:
receiving, by the Al learning part, a first medical image; generating, by the Al learning part, a second medical image from the first medical image by learning, the domain of the second medical image being different from that of the first medical image; or
first learning, wherein if the first medical image is one of a pair of paired data, the Al learning part compares a difference between the second medical image and another of the pair of paired data and performs the learning to reduce the difference.

According to embodiments, the paired data may include images belonging to a pair of different domains of a same region of a same patient or images belonging to a pair of different domains of same regions of different patients.

According to embodiments, the medical image conversion method may include, after generating the second medical image, regenerating, by the Al learning part, a third medical image from the second medical image by the learning, the domain of the third medical image being different from that of the second medical image. Here, the first medical image and the third medical image may belong to a same domain.

According to embodiments, the medical image conversion method may include second learning, wherein the Al learning part compares a difference between the first medical image and the third medical image and performs the learning to reduce the difference.

According to embodiments, the medical image conversion method may include, if the first medical image has both a first slice for a target region and a second slice for a non-target region, processing the first medical image so that the second slice is removed and the first slice remains before receiving the first medical image.

According to embodiments, the first medical image may include lesion data and normal data.

According to embodiments, the medical image conversion method may include, if the lesion data is smaller than the normal data, amplifying the lesion data. According to embodiments, both the lesion data and the normal data may be defined in a slice unit. For example, a slice including a lesion region may be defined as lesion data, and a slice including only normal tissue may be defined as normal data. Here, the slice may include each tomogram acquired when tomography is performed along the longitudinal direction in succession.

According to embodiments, the operation of amplifying the lesion data includes replicating the lesion data so that the ratio of the lesion data may be equal to the ratio of the normal data. In this context, the duplication may include simply copying.

According to another aspect of the disclosure, provided is a computer-readable recording medium having recorded thereon a program for executing the medical image conversion method described above.

### Advantageous Effects

According to embodiments of the disclosure as set forth above,
a medical image conversion method and device for performing conversion between medical images belonging to different domains may be provided. For example, a medical image conversion method and device able to generate an MR image based on a CT image and, conversely, a CT image based on an MR image may be provided.

Furthermore, an Al learning model which performs conversion between medical images belonging to different domains may be provided. For example, an Al learning model able to generate an MR image based on a CT image and, conversely, a CT image based on MR an image may be provided.

In addition, the disclosure has a variety of effects with excellent versatility depending on the embodiment, and such effects may be clearly understood from the following description of embodiments.

### DESCRIPTION OF DRAWINGS

The following drawings accompanying the specification illustrate embodiments of the disclosure and, together with the foregoing disclosure, serve to provide further understanding of the disclosure, and thus, the disclosure should not be construed as being limited to the drawings, wherein:
FIG. 1 illustrates a first embodiment of a medical image conversion method according to embodiments of the disclosure.
FIG. 2 illustrates a second embodiment in which amplifying lesion data is added to the first embodiment of FIG. 1.
FIG. 3 illustrates a third embodiment in which an operation of processing a first medical image to remove a second slice so that only a first slice remains is added to the first embodiment.
FIG. 4 illustrates a fourth embodiment in which an operation of calculating a match rate is added to the first embodiment of FIG. 1.

### BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

Terms as used herein are used to describe a particular embodiment and are not intended to limit the disclosure. Singular forms are intended to include plural forms, unless the context clearly indicates otherwise.

Terms, such as "include" or "have", or the like, as used herein indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar constituent elements are given the same reference numerals regardless of the reference numerals of the drawings, and repeated descriptions thereof will be omitted.

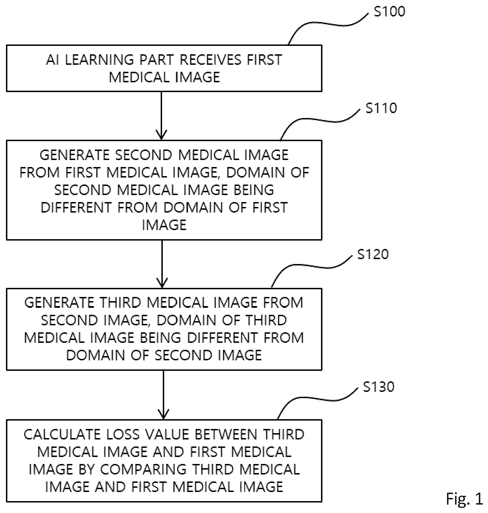

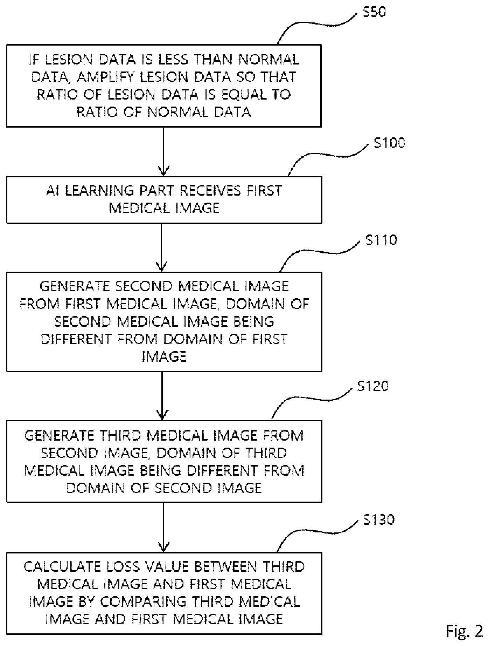

FIG. 1 illustrates a first embodiment of a medical image conversion method according to embodiments of the disclosure, and FIG. 2 illustrates a second embodiment in which amplifying lesion data is added to the first embodiment of FIG. 1.

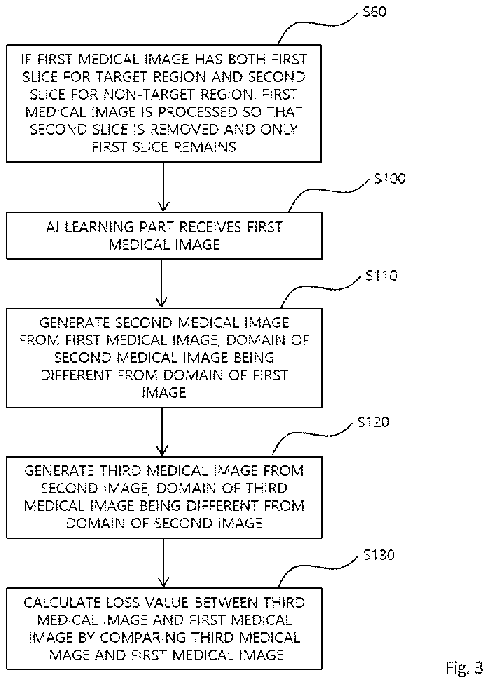

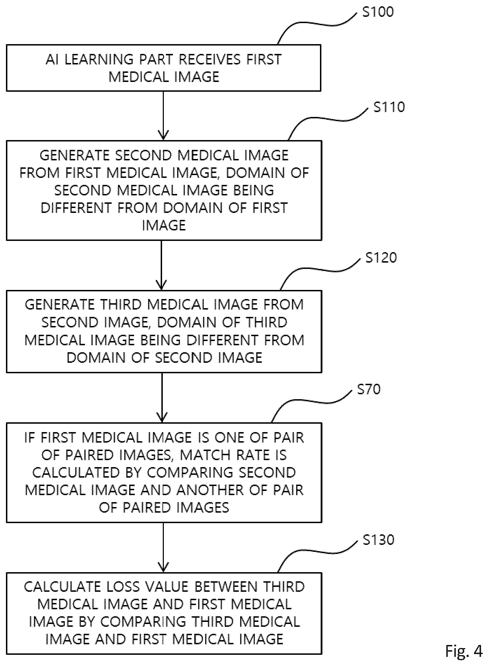

FIG. 3 illustrates a third embodiment in which an operation of processing a first medical image to remove a second slice so that only a first slice remains is added to the first embodiment, and FIG. 4 illustrates a fourth embodiment in which an operation of calculating a match rate is added to the first embodiment of FIG. 1.

According to embodiments of the disclosure, a method in which an artificial intelligence (AI) learning part performs conversion between medical images of different domains while performing learning may be presented.

The medical image conversion method according to embodiments may include: an operation S100 of receiving, by the Al learning part, a first medical image; an operation S110 of generating a second medical image from the first medical image, the domain of the second medical image being different from the domain of the first medical image;
an operation S120 of generating a third medical image from the second medical image, the domain of the third medical image being different from the domain of the second medical image; and an operation S130 of calculating a loss value between the third medical image and the first medical image by comparing the third medical image and the first medical image.

The first medical image may include an image obtained by an image capturing device (or imaging device). The image capturing device may include, for example, a computed tomography (CT) device or a magnetic resonance imaging (MRI) device (not shown). Such a CT device or MRI device may be used to capture a non-enhanced CT image, a non-enhanced MR image, a contrast enhanced CT, a contrast enhanced MR image, a T1 image, or a T2 image from a specific region of a patient's body.

In the disclosure, CT imaging and MR imaging may mean image reconstruction by computing in addition to optical meaning. However, the medical image herein does not exclude an image having an optical meaning.

In this context, the specific region of a patient may mean a specific region of the same patient. If the captured parts are the same, such regions may refer to specific regions of different patients. The image capturing device may capture a non-enhanced CT image, a non-enhanced MR image, a contrast enhanced CT image, a contrast enhanced MR image, a T1 image, or a T2 image of the specific region of the patient.

For example, the non-enhanced CT image or the non-enhanced MR image captured here may be an input image or a target image.

Here, input and target images may refer to a pair of paired data. For example, a CT image and an MR image captured from the same specific region of the same patient may be a pair of paired data. A CT image and an MR image captured from the same regions of different patients may also be a pair of paired data.

For example, the contrast enhanced CT image and the non-enhanced CT image of the same part of a subject, i.e., a subject from which an image is to be captured, may be a pair of paired data. The contrast enhanced MR image and the non-enhanced MR image of the same part may also be a pair of paired data, and a T1 image and a T2 image of the same area may also be paired data.

If the non-enhanced CT image is the input image, the input non-enhanced CT image and the contrast enhanced CT image, which is the paired data, may be the target images. If the non-enhanced MR image is the input image, the input non-enhanced MR image and the contrast enhanced CT image, which is the paired data, may be the target images.

If the contrast enhanced CT image is the input image, the contrast enhanced CT image, which is the input image, and the non-enhanced CT image, which is the paired data, may be the target image.

If the contrast-enhanced MR image is the input image, the contrast-enhanced MR image as the input image and the non-enhanced MR image as the paired data may be the target image. These input and target images may generally include an original image.

The meaning of different domains described herein will be explained. For example, the CT image and the MR image belong to different domains within the meaning of the disclosure, and non-enhanced images and contrast-enhanced images belong to different domains. The T1 image and the T2 image are equally MR images, but also belong to different domains within the meaning of the disclosure. An ultrasound image and the CT image belong to different domains. Similarly, the ultrasound image and the MR image belong to different domains. The MR image and a PET image belong to different domains. That is, one and another of the CT image, the MR image, the contrast enhanced image, the non-enhanced image, the T1 image, the T2 image, the PET image, or a SPECT image belong to different domains as described herein.

In other words, among the images realized by reconstruction based on the signal provided from the subject, if there is a difference in the signals or the sequences on which the image realization is based or in the image realization processes, the images having such a difference may be said to belong to different domains.

To describe the medical image conversion method according to embodiments of the disclosure, if an imaging modality acquires a pair of paired data by acquiring a CT image and an MR image from the same region of the same patient is taken as an example. In this case, if the CT image is determined to be the input image among the CT image and the MR image, which is the pair of paired data, the MR image may be determined to be the target image. In this case, the CT image and the MR image may be original images.

If the CT image is determined to be the input image, the CT image determined to be the input image in the medical image conversion method according to this embodiment may be a first medical image.

In other words, the Al learning part may be subjected to the operation of receiving the CT image as the first medical image. After receiving the first medical image, the Al learning part may generate a second medical image from the first medical image, in which the domain of second medical image is different from the first medical image. Here, the second medical image may mean an image generated based on the first medical image. For example, the MR image may be generated based on the CT image, which is the input image. In this case, the generated MR image may be a second medical image.

Here, in some embodiments, the operation of generating, by the Al learning part, the second medical image having a different domain from the first medical image, such as generating an MR image from a CT image, may be performed in the following two operations. However, the disclosure is not intended to be limited to the following two operations.

The first operation may include one or more layers which receive two-dimensional or three-dimensional image data acquired from the image capturing device or the like and perform three-dimensional convolutional operations to extract high-dimensional features having smaller sizes than the original image. Such layers may be represented by a feature extraction artificial neural network. Such a feature extraction artificial neural network may receive two-dimensional or three-dimensional real-world image data captured by the image capturing device and perform three-dimensional convolutional operations to extract high-dimensional image features having smaller sizes than the original image.

The second operation may include one or more layers which perform a three-dimensional (3D) deconvolution operation for the purpose of generating two-dimensional or three-dimensional image data of a different modality from the result of the first operation. Such layers may be represented by an artificial neural network which generates an image of a different modality. The artificial neural network which generates the different modality image may generate the two-dimensional or three-dimensional different modality image data by performing a three-dimensional (3D) deconvolution operation from the result of the three-dimensional convolutional operation of the artificial neural network which extracts the features in the first operation.

The medical image conversion method according to this embodiment may include, after generating the third medical image, an operation S130 of calculate the loss value between the generated third medical image and the first medical image by comparing the two images. For example, if the CT image is the first medical image in the above example, the MR image synthesized based on the CT image may be the second medical image, and a resynthesized CT image may be generated based on the synthesized MR image. Here, the resynthesized CT image may be the third medical image.

In some embodiments, the first medical image and the third medical image may be images belonging to the same domain. That is, if the first medical image is a CT image, the third medical image may also be a CT image. For example, if the first medical image is an MR image, the third medical image may also be an MR image.

In some embodiments, the first medical image and the third medical image may be images belonging to the same domain. In this case, the resynthesized CT image, which is the third medical image in the above example, is desired to be indistinguishable from the CT image, which is the first medical image (i.e., input image). The Al learning part may calculate the accuracy of the image conversion by comparing the third medical image and the first medical image and calculate the loss value between the two images.

In some embodiments, the operation S130 of calculating the loss value, the Al learning part may perform the calculation of the loss value repeatedly in each iteration when performing learning. For example, the Al learning part may calculate the loss value between a regenerated image and the input image in each iteration while performing the learning. That is, in the example above, the loss value between the CT image and the reconstructed CT image may be calculated.

In some embodiments, in a case in which the iteration includes a first iteration and a second iteration which are sequential, if the absolute value of the change between a first loss value calculated in the first iteration and a second loss value calculated in the second iteration is less than a threshold value, the Al learning part may regard that overfitting has occurred and stop the learning.

The Al learning is not only tailored to the distribution of the given learning data, but the Al learning may be completed by not proceeding after the required medical images have been sufficiently learned. The Al learning part which has been trained as above may now be applied to medical images having different data distributions.

In some embodiments, in the operation S100 of receiving the first medical image, the first medical image may include lesion data and normal data. For example, if the CT image is the first medical image among the pair of paired data of the CT image and the MR image captured by the image capturing device, the CT image may include both lesion data and normal data. Here, the lesion data may refer to a region where a lesion is present, the and normal data may refer to a region which is normal other than the region where the lesion is present. The lesion data and the normal data may exist together if the image is captured from the same region of the same patient, and may also exist together if the images are captured from the same regions of different patients.

In general, the lesion data has a smaller area than the normal data, and if images are captured from a plurality of patients, the number of data having lesions is small. Therefore, the normal data is usually overwhelmingly larger than the lesion data.

If the lesion data is less than the normal data in the operation S100 of receiving the first medical image, the operation S50 of amplifying the lesion data so that the ratio of the lesion data is equal to the ratio of the normal data may be performed before the operation S100 of receiving the first medical image.

In the operation S50 of amplifying the lesion data, for example, if the number of normal data is 1,000 and the number of lesion data is 10, the lesion data may be further replicated 100 times so that the number of lesion data is 1,000, thereby causing the ratio between the number of normal data and the number of lesion data to be 1:1, and then the lesion data may be used as input data.

If the learning based on normal data is superior to the learning based on lesion data, the Al learning part may not achieve accurate learning, and the accuracy of the result of the medical image conversion may not be improved unless the same amount of lesion data as normal data is learned.

In some embodiments, if the first medical image has both a first slice for a target region and a second slice for a non-target region before the operation S100 of receiving the first medical image, an operation S60 of processing the first medical image may be performed so that the second slice is removed and only the first slice remains.

To receive only a part to be learned by the Al learning part from the first medical image, the operation of removing a part which is unnecessary for the learning from the first medical image before the first medical image is input to the Al learning part may additionally be performed before the operation S100 of receiving the first medical image.

An object to be learned by the Al learning part, i.e., the target region, may be, for example, the brain or the liver. If the target region is the brain, the image capturing device which captures CT images of the brain continuously captures a plurality of tomograms along the longitudinal direction while generating a plurality of slices, and thus the scope of image capturing may range from the tip of the head to the knees, not just the brain.

In this case, to register the input CT image with the target MR image, it is necessary to use only slices corresponding to the brain region (i.e., the target region) in the CT image. In some embodiments, the method of extracting only the target region may use an image intensity thresholding algorithm or the like.

According to this embodiment, the Al may eliminate input data which is unnecessary for the learning process in advance to significantly reduce the learning time.

In some embodiments, if one of the first medical image or the second medical image, which belongs to a different domain from the first medical image and is captured from the same region as the first medical image, is input as the first medical image, an operation S70 of calculating a match rate by comparing the second medical image and a second original image may be further included.

The pair of paired data may include a first source image and a second source image. The second source image may be an image captured from the same region as the first source image.

Here, the first source image and the second source image are not desired to be from the same patient.

For example, if the first original image is a CT image and the second original image is an MR image, the method may further include an operation S70 of calculating the match rate by comparing an MR image, which is the second medical image generated based on the CT image, and an MR image, which is the second original image, when the CT image is input as the first medical image.

In some embodiments, the match rate may be calculated using the Hausdorff distance or the Dice similarity coefficient, and if the second medical image and the other of the pair of paired data each contain a lesion region, the match rate may be calculated quantitatively using the difference in the center of mass of the locations of the two lesions.

According to this embodiment, in addition to the operation S130 of calculating the loss value by comparing the third medical image, which is the reconstructed medical image, and the first medical image, the accuracy of the medical image conversion may be improved by measuring the accuracies of the respective results if the second medical image is generated and if the third medical image is generated by calculating the match rate by comparing the generated medical image, i.e., the second medical image, with the first medical image and the paired data medical image.

Other embodiments of the disclosure may present a computer-readable recording medium having recorded thereon a program for executing the above-described medical image conversion method.

According to another aspect of the disclosure, a sub-input image containing asymmetric information of the original medical image may be received as an input together with the original medical image, which is the image input to the Al learning part. That is, the Al learning part may receive paired data of the original medical image and the sub-input image as an input.

The asymmetric information as used herein may include a resultant image obtained by calculating a difference in voxel values regarding the original medical image and after left-right inverting the original medical image and then fitting the left-right inverted image to the original medical image by the non-rigid transform technique.

For example, the sub-input image may include a resultant image C. If an original CT image A is received as input data, the Al learning part produces resultant image C by left-right inverting the original CT image A, fitting the original CT image A and the left-right inverted CT image B, and then calculating the difference in voxel values between the original CT image A and the left-right inverted CT image B. Here, the resultant image C may be the sub-input image.

In this case, the original CT image A and the resultant image C may be paired data, and may be input to the Al learning part as a pair of input images.

CT images of lesions from strokes, brain tumors, or the like may show small differences in intensity, but such differences may be difficult to detect with the naked eye. Because these lesions are often asymmetrically distributed from side to side, the difference in voxel values between the left and right sides may be used to highlight the region where the lesion is located.

By using the original CT image A and the resultant image C as images input to the Al learning part, an image in which the region having the lesion is highlighted may be obtained.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware such as ROMs, RAMs, and flash memories specifically configured to store program instructions and execute the program instructions.

In addition, the computer programs may be specifically designed and configured for the disclosure or may be known and available to a person having ordinary knowledge in the art of computer software. Examples of computer programs may include machine code produced by compilers and high-level language code executable on computers using interpreters.

In the specification of the disclosure, the use of the term "the" or similar denoting terms may correspond to both singular and plural forms.

Furthermore, recitation of ranges of values herein are merely intended to refer to respective separate values falling within the respective ranges and (unless otherwise indicated herein) the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the disclosure shall not be limited to the above-described embodiments, and the entire scope of the appended claims and equivalents thereof will fall within the scope of the disclosure.

### <Description of Reference Numerals of Drawings>

S100: operation of receiving first medical image
S110: operation of generating second medical image
S120: operation of generating third medical image
S130: operation of calculating loss value

## Claims

1. A computer-implemented medical image conversion method in which an artificial intelligence (AI) learning part performs conversion between medical images of different domains while performing learning, the medical image conversion method comprising:
receiving, by the Al learning part, a first medical image;
generating, by the Al learning part, a second medical image from the first medical image by learning, the domain of the second medical image being different from the domain of the first medical image;
regenerating, by the Al learning part, a third medical image from the second medical image by the learning, a domain of the third medical image being different from the domain of the second medical image, wherein the first medical image and the third medical image belong to a same domain;
first learning, wherein if the first medical image is one of a pair of paired data, the Al learning part compares a difference between the second medical image and another of the pair of paired data and performs the learning to reduce the difference,
wherein, if the first medical image is one of the pair of paired data, a match rate of the second medical image and the other of the pair of paired data is calculated by comparing the second medical image with the other of the pair of paired data; and
wherein, if the second medical image and the other of the pair of paired data each contain a lesion region, the match rate is calculated quantitatively using a difference in locations of the two lesions, including a difference in a center of mass of the locations of the two lesions.

2. The medical image conversion method of claim 1, wherein the paired data comprise images belonging to a pair of different domains of a same region of a same patient or images belonging to a pair of different domains of same regions of different patients.

3. The medical image conversion method of claim 1, comprising second learning, wherein the Al learning part compares a difference between the first medical image and the third medical image and performs the learning to reduce the difference.

4. The medical image conversion method of claim 1, comprising, if the first medical image has both a first slice for a target region and a second slice for a non-target region, processing the first medical image so that the second slice is removed and the first slice remains before receiving the first medical image.

5. The medical image conversion method of claim 1, wherein the first medical image comprises lesion data and normal data.

6. The medical image conversion method of claim 5, comprising, if the lesion data is smaller than the normal data, amplifying the lesion data.

7. The medical image conversion method of claim 6, wherein amplifying the lesion data comprises replicating the lesion data so that the ratio of the lesion data is equal to the ratio of the normal data.

8. A computer-readable recording medium having recorded thereon a program which, when executed by a computer, causes the computer to perform the computer-implemented medical image conversion method according to any one of claims 1 to 7.

## Patentansprüche

1. Computerimplementiertes Verfahren zur Umwandlung medizinischer Bilder, bei dem eine Lerneinheit für künstliche Intelligenz (KI) während des Lernens eine Umwandlung zwischen medizinischen Bildern unterschiedlicher Domänen durchführt, wobei das Verfahren zur Umwandlung medizinischer Bilder umfasst:
Empfangen eines ersten medizinischen Bildes durch die Kl-Lerneinheit;
Erzeugen eines zweiten medizinischen Bildes aus dem ersten medizinischen Bild durch die Kl-Lerneinheit mittels Lernens, wobei die Domäne des zweiten medizinischen Bildes von der Domäne des ersten medizinischen Bildes verschieden ist;
erneutes Erzeugen eines dritten medizinischen Bildes aus dem zweiten medizinischen Bild durch die KI-Lerneinheit mittels des Lernens, wobei die Domäne des dritten medizinischen Bildes von der Domäne des zweiten medizinischen Bildes verschieden ist und wobei das erste medizinische Bild und das dritte medizinische Bild derselben Domäne angehören;
eine erste Lernstufe, wobei, wenn das erste medizinische Bild eines der Daten eines Paars gepaarter Daten ist, die KI-Lerneinheit eine Differenz zwischen dem zweiten medizinischen Bild und dem anderen Datenelement des Paars gepaarter Daten vergleicht und das Lernen derart durchführt, dass die Differenz verringert wird,
wobei, wenn das erste medizinische Bild eines der Daten des Paars gepaarter Daten ist, ein Übereinstimmungsgrad zwischen dem zweiten medizinischen Bild und dem anderen Datenelement des Paars gepaarter Daten berechnet wird, indem das zweite medizinische Bild mit dem anderen Datenelement des Paars gepaarter Daten verglichen wird; und
wobei, wenn das zweite medizinische Bild und das andere Datenelement des Paars gepaarter Daten jeweils einen Läsionsbereich enthalten, der Übereinstimmungsgrad quantitativ unter Verwendung einer Differenz zwischen den Positionen der beiden Läsionen, einschließlich einer Differenz zwischen den Positionen der Massenschwerpunkte der beiden Läsionen, berechnet wird.

2. Verfahren zur Umwandlung medizinischer Bilder nach Anspruch 1, wobei die gepaarten Daten Bilder umfassen, die einem Paar unterschiedlicher Domänen derselben Region desselben Patienten angehören, oder Bilder, die einem Paar unterschiedlicher Domänen derselben Regionen unterschiedlicher Patienten angehören.

3. Verfahren zur Umwandlung medizinischer Bilder nach Anspruch 1, umfassend eine zweite Lernstufe, wobei die KI-Lerneinheit eine Differenz zwischen dem ersten medizinischen Bild und dem dritten medizinischen Bild vergleicht und das Lernen derart durchführt, dass die Differenz verringert wird.

4. Verfahren zur Umwandlung medizinischer Bilder nach Anspruch 1, umfassend, wenn das erste medizinische Bild sowohl eine erste Schicht für eine Zielregion als auch eine zweite Schicht für eine Nichtzielregion aufweist, das Verarbeiten des ersten medizinischen Bildes vor dem Empfangen des ersten medizinischen Bildes derart, dass die zweite Schicht entfernt wird und die erste Schicht verbleibt.

5. Verfahren zur Umwandlung medizinischer Bilder nach Anspruch 1, wobei das erste medizinische Bild Läsionsdaten und Normaldaten umfasst.

6. Verfahren zur Umwandlung medizinischer Bilder nach Anspruch 5, umfassend, wenn die Menge der Läsionsdaten geringer als die Menge der Normaldaten ist, das Verstärken der Läsionsdaten.

7. Verfahren zur Umwandlung medizinischer Bilder nach Anspruch 6, wobei das Verstärken der Läsionsdaten das Replizieren der Läsionsdaten derart umfasst, dass der Anteil der Läsionsdaten dem Anteil der Normaldaten entspricht.

8. Computerlesbares Aufzeichnungsmedium, auf dem ein Programm aufgezeichnet ist, das bei Ausführung durch einen Computer den Computer veranlasst, das computerimplementierte Verfahren zur Umwandlung medizinischer Bilder nach einem der Ansprüche 1 bis 7 durchzuführen.

## Revendications

1. Procédé de conversion d'images médicales mis en œuvre par ordinateur, dans lequel une unité d'apprentissage d'intelligence artificielle (IA) effectue, au cours d'un apprentissage, une conversion entre des images médicales appartenant à des domaines différents, le procédé comprenant :
la réception, par l'unité d'apprentissage d'IA, d'une première image médicale ;
la génération, par l'unité d'apprentissage d'IA, d'une deuxième image médicale à partir de la première image médicale au moyen de l'apprentissage, le domaine de la deuxième image médicale étant différent du domaine de la première image médicale ;
la régénération, par l'unité d'apprentissage d'IA, d'une troisième image médicale à partir de la deuxième image médicale au moyen de l'apprentissage, le domaine de la troisième image médicale étant différent du domaine de la deuxième image médicale, la première image médicale et la troisième image médicale appartenant à un même domaine ;
un premier apprentissage dans lequel, si la première image médicale est l'une des données d'une paire de données appariées, l'unité d'apprentissage d'IA compare une différence entre la deuxième image médicale et l'autre donnée de la paire de données appariées et effectue l'apprentissage de manière à réduire cette différence,
dans lequel, si la première image médicale est l'une des données de la paire de données appariées, un taux de correspondance entre la deuxième image médicale et l'autre donnée de la paire de données appariées est calculé en comparant la deuxième image médicale à l'autre donnée de la paire de données appariées ; et
dans lequel, si la deuxième image médicale et l'autre donnée de la paire de données appariées contiennent chacune une région de lésion, le taux de correspondance est calculé quantitativement à l'aide d'une différence entre les positions des deux lésions, comprenant une différence entre les positions des centres de masse des deux lésions.

2. Procédé de conversion d'images médicales selon la revendication 1, dans lequel les données appariées comprennent des images appartenant à une paire de domaines différents d'une même région d'un même patient, ou des images appartenant à une paire de domaines différents de mêmes régions de patients différents.

3. Procédé de conversion d'images médicales selon la revendication 1, comprenant un second apprentissage dans lequel l'unité d'apprentissage d'IA compare une différence entre la première image médicale et la troisième image médicale et effectue l'apprentissage de manière à réduire cette différence.

4. Procédé de conversion d'images médicales selon la revendication 1, comprenant, si la première image médicale comporte à la fois une première coupe correspondant à une région cible et une deuxième coupe correspondant à une région non cible, le traitement de la première image médicale, avant la réception de la première image médicale, de manière à supprimer la deuxième coupe et à conserver la première coupe.

5. Procédé de conversion d'images médicales selon la revendication 1, dans lequel la première image médicale comprend des données de lésion et des données normales.

6. Procédé de conversion d'images médicales selon la revendication 5, comprenant, si les données de lésion sont moins nombreuses que les données normales, l'amplification des données de lésion.

7. Procédé de conversion d'images médicales selon la revendication 6, dans lequel l'amplification des données de lésion comprend la réplication des données de lésion de sorte que la proportion des données de lésion soit égale à celle des données normales.

8. Support d'enregistrement lisible par ordinateur sur lequel est enregistré un programme qui, lorsqu'il est exécuté par un ordinateur, amène l'ordinateur à mettre en oeuvre le procédé de conversion d'images médicales mis en oeuvre par ordinateur selon l'une quelconque des revendications 1 à 7.
